# EUROPEAN PATENT SPECIFICATION

(11) **EP 4 440 599 B1**
(45) Date of publication and mention of the grant of the patent: **25.02.2026**
(21) Application number: 23700010.4
(22) Date of filing: 03.01.2023
(51) Int. Cl.: A61K 38/20, A61K 47/68, A61P 35/00, A61K 31/519

(54) **COMBINATION OF AN IMMUNOCYTOKINE COMPRISING IL-12 AND A KINASE INHIBITOR**
KOMBINATION EINES IMMUNZYTOKINS AUFWEISEND IL-12 UND EINEM KINASEINHIBITOR
COMBINAISON D'UNE IMMUNOCYTOKINE COMPRENANT DE L'IL-12 ET UN INHIBITEUR DE KINASE

(30) Priority: 04.01.2022 EP 22150233
(43) Date of publication of application: 09.10.2024
(73) Proprietor: Philogen S.p.A., 53100 Siena (IT)
(72) Inventor: DAKHEL PLAZA, Sheila, 8112 Otelfingen (CH); ROTTA, Giulia, 8112 Otelfingen (CH); NERI, Dario, 53018 Sovicille ( SI) (IT)
(74) Representative: Michalski Hüttermann & Partner Patentanwälte mbB
(86) International application number: PCT/EP2023/050081
(87) International publication number: WO 2023/131611

(56) References cited:
- EP-A2- 2 467 165
- WO-A1-2021/209452
- US-A1- 2020 397 915
- THOMAS LIST ET AL: "Immunocytokines: a review of molecules in clinical development for cancer therapy", CLINICAL PHARMACOLOGY: ADVANCES AND APPLICATIONS, 1 August 2013 (2013-08-01), pages 29 - 45, XP055289369, DOI: 10.2147/CPAA.S49231
- HU XIAOYI ET AL: "The JAK/STAT signaling pathway: from bench to clinic", vol. 6, no. 1, 1 December 2021 (2021-12-01), XP055925222, Retrieved from the Internet <URL:https://www.nature.com/articles/s41392-021-00791-1.pdf> DOI: 10.1038/s41392-021-00791-1
- NGUYEN KHUE G. ET AL: "Localized Interleukin-12 for Cancer Immunotherapy", FRONTIERS IN IMMUNOLOGY, vol. 11, 1 January 2020 (2020-01-01), pages 575597, XP055925153, DOI: 10.3389/fimmu.2020.575597

## Description

### Field of the invention

The invention relates to the field of immunoconjugates.

### Background

Cytokines are key mediators of innate and adaptive immunity. Many cytokines have been used for therapeutic purposes in patients with advanced cancer, but their administration is typically associated with severe toxicity, hampering dose escalation to therapeutically active regimens and their development as anticancer drugs. To overcome these problems, the use of 'immunocytokines' (i.e. cytokines fused to antibodies or antibody fragments) has been proposed, with the aim to concentrate the immune-system stimulating activity at the site of disease while sparing normal tissues (Neri & Bicknell, 2005). However, genetically fusing a cytokine to an antibody or to an antibody fragment creating an "immunocytokine", does not always result in an immunocytokine that retains the ability to target the tumor of the antibody. For example, in certain Interleukin-7 fusions (Pasche et al. (2011) J Biotechnology, 154, 84-92) the tumor targeting was completely abrogated, while in certain GM-CSF fusions (Kaspar et al. (2007) Cancer Res, 67, 4940-4948) the tumor targeting ability was found to be dose dependent.

IL-12 is produced by antigen presenting cells, such as macrophages and CDlc + Dendritic Cells, and acts upon Natural Killer (NK) cells, CD8⁺Cytotoxic T cells, and CD4⁺ T helper cells. Originally called Natural Killer cell stimulating factor, IL12 promotes the cytotoxic activity of NK cells and CD8⁺ T cells and promotes polarization of CD4⁺ T cells towards a type 1 phenotype.

Interestingly, human CD4⁺ and CD8⁺ T cells introduced into the xenogeneic environment of humanized mice without IL-12 preferentially differentiate into type 2 (IL4+ GATA3+) or mixed type 1 and 2 (IFNG+ TBET+ IL4+ GATA3+) subsets. Injection of recombinant human IL-12 in mice was able restore differentiation towards a type 1 to improve cytotoxic immunity to a viral challenge. In humans, genetic mutations in IL-12p40 and one component of the IL-12 receptor, IL-12RB1, have been observed in patients with recurrent mycobacterial disease, suggestive of insufficient type 1 cell-mediated immunity. In mice, genetic deletion of other component of the IL-12 receptor, IL-12RB2, increases susceptibility to spontaneous autoimmunity, B-cell malignancies, and lung carcinomas.

As a single agent, intravenous injection of recombinant IL-12 exhibited modest clinical efficacy in a handful of patients with advanced melanoma and renal cell carcinoma. However, one death due to *Clostridia perfringens* septicemia in the first Phase I study limits interest in the systemic delivery of IL-12.

As a combination therapy, IL-12 has been used as an adjuvant to enhance cytotoxic immunity using a melanoma antigen vaccine or using peptide-pulsed peripheral blood mononuclear cells and to promote NK-cell mediated killing of HER2 -positive breast cancer cells in patients treated with trastuzumab.

Just like many other cytokines, the administration of recombinant human IL-12 is associated with severe toxicity, hampering its development as an anticancer drug.

Clinical trials in patients with cancer have revealed promising therapeutic activities but have also shown that recombinant human IL-12 is extremely toxic to humans, with a maximal tolerated dose of 0.5 µg/kg of body weight.

The toxic side effects of toxins, particularly cytokines such as such as IL-12 have made it difficult to administer an effective dose and to reach high concentrations at the site of a tumour.

Previously, researchers have attempted to overcome these drawbacks by targeted delivery of IL-12 cytokine to the tumour environment through, e.g., conjugation to antibodies specific for antigens associated to cancer growth. These cytokine - antibody conjugates are often referred to as "immunocytokines"

Current applicants have shown that the therapeutic index of the IL-12 cytokine can be improved by conjugation to antibody fragments such as scFv antibody fragments (WO2006/119897) or to single-chain diabodies (WO2013/014149).

Other versions of targeted IL-12 such as L19-IL12 with improved linkers (WO2019/122025, WO2021/209452) have also been reported by the current applicants.

US2020/397915A1 refers to a targeted immunocytokine comprising IL-12 linked to a targeting moiety, i.e. an anti-fibronectin antibody, for use in treating cancer.

EP2467165A2 refers to an immunoconjugate IL-12 - L19, comprising IL-12 linked to a targeting moiety, i.e. an anti-fibronectin antibody, for use in treating cancer.

Thomas List et al. (2013) refers to immunocytokine IL-12 fused to L19 antibody, and describes that a therapeutic effect could be achieved with lower doses compared to free IL-12.

Hu Xiaoyi et al. (2021) refers to immunocytokine IL-12 fused to BC1 antibody, and describes that Janus kinase inhibitors such as ruxolitinib are known for use in treating cancer.

Nguyen Khue et al. (2020) refers to two immunocytokines, huBC1.IL12 and IL12-L19 and describes that IL12-L19 enhances cytotoxic T and NK cell activity while reversing tumor-induced immunosuppression.

However, the need to improve the therapeutic index of targeted IL-12 remains. For example, it would be highly advantageous to find a way to decrease the IL-12 related toxicity while maintaining its anti-cancer efficacy.

To overcome the drawbacks associated with IL-12 therapy, delivery of IL-12 to the tumor site by means of an antibody directed against tumor-associated marker to increase local concentrations of IL-12 at the tumour site, as well as reduce toxicities associated with systemic administration of IL-12 has been proposed. In particular, the concentration of cytokines at the level of tumour blood vessels is an attractive therapeutic strategy as the tumour neovasculature is more accessible to intravenously administered therapeutic agents than tumour cells, which helps avoid problems associated with the interstitial hypertension of solid tumours. In addition, angiogenesis is characteristic of most aggressive solid tumours. Angiogenesis describes the growth of new blood vessels from existing blood vessels. Tumours can induce angiogenesis through secretion of various growth factors (e.g. Vascular Endothelial Growth Factor). Tumour angiogenesis allows tumours to grow beyond a few millimetres in diameter and is also a prerequisite for tumour metastasis. New blood vessels formed as the result of angiogenesis form the neovasculature of the tumour or the tumour metastases. Targeting IL-12 to the neovasculature should allow the immunotherapy of a variety of different tumour types.

The alternatively spliced extra domain B (ED-B) of fibronectin represent one of the best-characterised markers of angiogenesis and has been reported to be expressed around the neovasculature and in the stroma of virtually all types of aggressive solid tumours. Furthermore, even non-solid cancers, such as leukaemia, may be amenable to treatment by targeting antigens of the neovasculature. WO2011/015333 described treating leukaemia, including acute myeloid leukaemia, by targeting the bone marrow neovasculature.

A human monoclonal antibody specific to this target named L19 has been extensively described (WO1999/058570, WO2003/076469, WO2005/023318).

In addition, immunocytokines based on L19 are currently being investigated in Phase I, Phase II and Phase III clinical trials in patients with cancer. These immunocytokines include several cytokines, comprising IL-12.

It is one object of the present invention to broaden the scope of therapeutic applications of the above identified immunocytokines.

It is another object of the present invention to provide new therapeutic options for conditions for which so far no adequate treatment option exists.

It is another object of the present invention to improve efficacy of a therapy using the above described immunocytokines.

### Brief description of the Figures

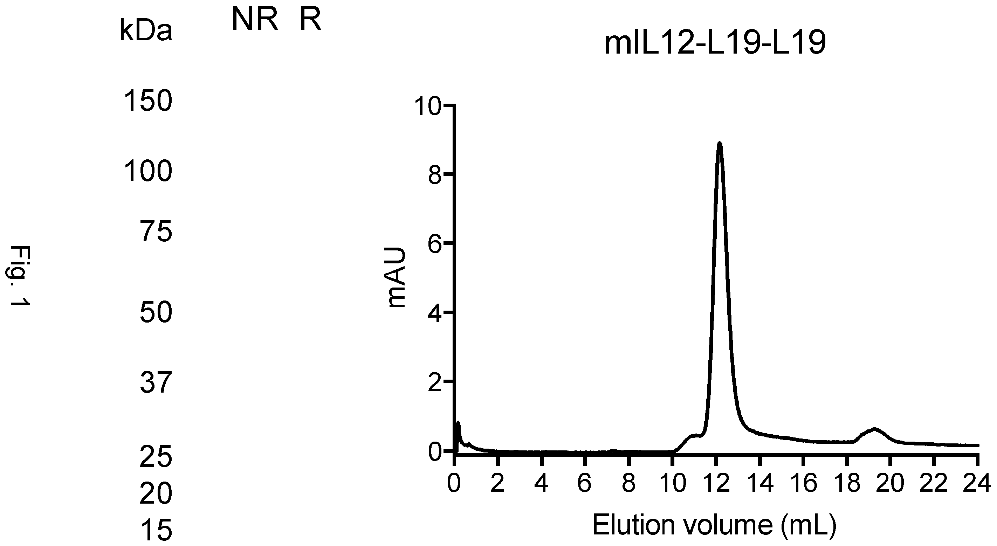

Fig. 1: SDS-PAGE analysis of purified L19-IL12 under nonreducing (NR) and reducing (R) conditions (left) and size-exclusion chromatography profile of L19-IL12 (right).
Fig. 2A: Antitumor activity in tumor bearing mice of (i) vehicle (ii) Ruxolitinib (iii) three different dosages of L19-IL12 administered alone or (iv) in combination with Ruxolitinib.
Fig. 2B: Toxicity expressed in body weight change of (i) vehicle (ii) Ruxolitinib 75 mg/kg (iii) L19-IL12 (0.6 mg/kg) alone or (iv) in combination with Ruxolitinib
Fig. 2C: Toxicity expressed in body weight change of (i) vehicle (ii) Ruxolitinib (iii) L19-IL12 (0.9 mg/kg) alone or (iv) in combination with Ruxolitinib
Fig. 2D: Toxicity expressed in body weight change of (i) vehicle (ii) Ruxolitinib (iii) L19-IL12 (1.2 mg/kg) alone or (iv) in combination with Ruxolitinib. The days of injection are represented by the black arrows.

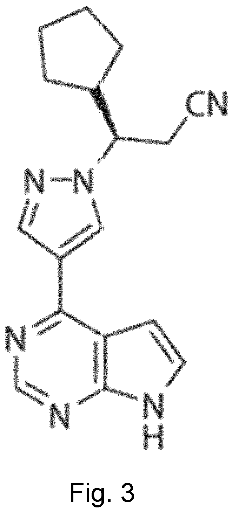

Fig. 3: Chemical structure depiction of Ruxolitinib.

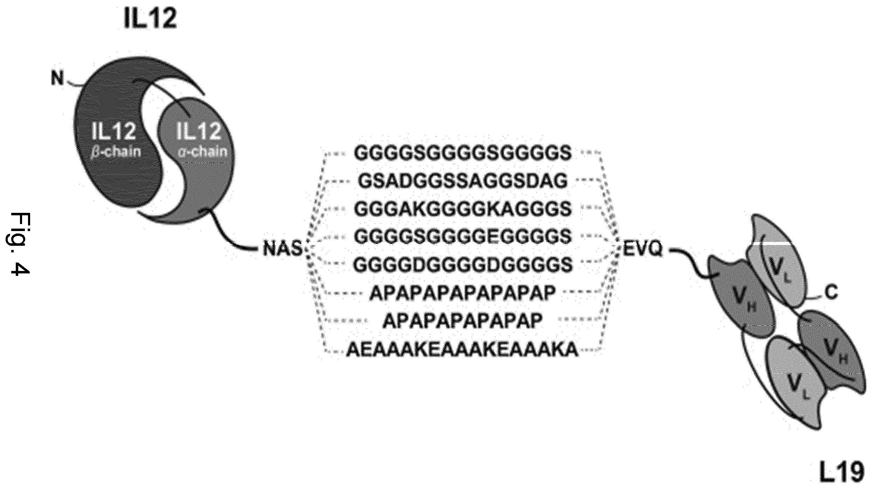

Fig. 4: Exemplary immunocytokine formats using IL-12 and an anti-fibronectin antibody.
Fig. 5: *In vitro* quantification of interferon-*γ* levels in human cells incubated with L19-IL12 and with different doses of JAK kinase inhibitors and Dexamethasone.
Fig. 6A: Antitumor activity in tumor bearing mice of (i) vehicle (ii) Ruxolitinib (iii) two high doses (2.4 mg/kg or 3.0 mg/kg) of L19-IL12 administered alone or (iv) in combination with Ruxolitinib.
Fig. 6B: Toxicity expressed in body weight change of (i) vehicle (ii) Ruxolitinib (iii) L19-IL12 (2.4 mg/kg or 3.0 mg/kg) alone or (iv) in combination with Ruxolitinib. The days of injection are represented by the black arrows.

### Summary of the Invention

The invention is defined by the features of the appended independent claims. Preferred embodiments are defined by the features of the dependent claims.

In particular, the present invention relates to a combination comprising at least
(a) a recombinant protein comprising
   (i) interleukin-12 (IL-12) and
   (ii) a targeting entity which comprises an antibody binding the extra-domain B (ED-B) of fibronectin, or a target binding fragment, and
(b) a kinase inhibitor,

wherein the kinase inhibitor is the JAK inhibitor Ruxolitinib, and
wherein the targeting entity is an antibody, or a fragment thereof able to bind to a given target with high specificity and affinity.

The present invention further relates to the above combination for use in the treatment of a in a human or mammalian patient
(i) being diagnosed for,
(ii) suffering from or
(iii) being at risk of developing
cancer.

The present inventors have unexpectedly recognised that kinase inhibitors reduce the toxicity of targeted IL-12 while leaving its anti-cancer activity unaltered.

In particular, the present inventors have found that when a targeted immunocytokine comprising interleukin-12 (IL-12) is administered in tumor bearing mice in combination with such kinase inhibitors, the targeted IL-12 maintains its anti-cancer activity but, at the same time, a remarkable reduction in its toxicity is obtained.

There was no reason to expect *a priori* a reduced toxicity of IL-12 by combination with kinase inhibitors, but the results are even more surprising, as demonstrated by the experimental results.

As used herein, the term "kinase inhibitor" (TKI) relates to a compound, mostly a small molecule, that inhibits tyrosine kinases. Tyrosine kinases are enzymes responsible for the activation of many proteins by signal transduction cascades. The proteins are activated by adding a phosphate group to the protein (phosphorylation), a step that TKIs inhibit. TKIs are typically used as anticancer drugs. TKIs operate by four different mechanisms: they can compete with adenosine triphosphate (ATP), the phosphorylating entity, the substrate or both or can act in an allosteric fashion, namely bind to a site outside the active site, affecting its activity by a conformational change. Recently TKIs have been shown to deprive tyrosine kinases of access to the Cdc37-Hsp90 molecular chaperone system on which they depend for their cellular stability, leading to their ubiquitylation and degradation. Signal transduction therapy can also be used for non-cancer proliferative diseases and for inflammatory conditions.

As used herein, the term "Immunocytokine" relates to fusion proteins consisting of a cytokine moiety fused to a targeting entity. Immunocytokine products, which are specific to tumor-associated antigens on the cell membrane, have the potential to bridge tumor cells and certain leukocytes (e.g., T cells, or NK cells), in analogy to what could be achieved by using bispecific antibodies. By contrast, immunocytokines which target tumor-associated extracellular matrix components (e.g., splice isoforms of fibronectin or of tenascin-C), are believed to mainly display a biological activity which results from the high-density anchoring of the cytokine moiety at the site of disease.

As used herein, the term "targeting entity" relates to a molecule that is able to bind to a given target with high specificity and affinity. Such targeting entities are for example antibodies, or fragments or derivatives thereof.

Interleukin 12 (IL-12) is an interleukin that is naturally produced by dendritic cells, macrophages, neutrophils, and human B-lymphoblastoid cells (NC-37) in response to antigenic stimulation. IL-12 is composed of a bundle of four alpha helices. It is a heterodimeric cytokine encoded by two separate genes, IL-12A (p35) and IL-12B (p40). The active heterodimer (referred to as 'p70'), and a homodimer of p40 are formed following protein synthesis.

IL-12 is involved in the differentiation of naive T cells into Th1 cells. It is known as a T cell-stimulating factor, which can stimulate the growth and function of T cells. It stimulates the production of interferon-gamma (IFN-γ) and tumor necrosis factor-alpha (TNF-α) from T cells and natural killer (NK) cells, and reduces IL-4 mediated suppression of IFN-γ. T cells that produce IL-12 have a coreceptor, CD30, which is associated with IL-12 activity.

IL-12 plays an important role in the activities of natural killer cells and T lymphocytes. IL-12 mediates enhancement of the cytotoxic activity of NK cells and CD8+ cytotoxic T lymphocytes. There also seems to be a link between IL-2 and the signal transduction of IL-12 in NK cells. IL-2 stimulates the expression of two IL-12 receptors, IL-12R-β1 and IL-12R-β2, maintaining the expression of a critical protein involved in IL-12 signaling in NK cells. Enhanced functional response is demonstrated by IFN-γ production and killing of target cells.

IL-12 also has anti-angiogenic activity, which means it can block the formation of new blood vessels. It does this by increasing production of interferon gamma, which in turn increases the production of a chemokine called inducible protein-10 (IP-10 or CXCL10). IP-10 then mediates this anti-angiogenic effect. Because of its ability to induce immune responses and its anti-angiogenic activity, there has been an interest in testing IL-12 as a possible anti-cancer drug. However, it has not been shown to have substantial activity in the tumors tested to this date. There is a link that may be useful in treatment between IL-12 and the diseases psoriasis & inflammatory bowel disease.

In some examples, the first subunit of the IL-12 protein is a p40 and the second subunit is a p35.

In some examples, the first subunit of the IL-12 protein is a p40 comprising an amino acid sequence having at least 75%, 80%, 85%, 90%, 91%, 92%, 93%, 94%, 95%, 96%, 97%, 98%, 99%, or 100% identity to the amino acid sequence set forth in SEQ ID NO: 1 or a fragment thereof, wherein the IL-12 protein can activate an IL-12 receptor.

In some examples, the second subunit of the IL-12 protein is a p35 comprising an amino acid sequence having at least 75%, 80%, 85%, 90%, 91%, 92%, 93%, 94%, 95%, 96%, 97%, 98%, 99%, or 100% identity to the amino acid sequence set forth in SEQ ID NO: 3 or a fragment thereof, wherein the IL-12 protein can activate an IL-12 receptor.

Further disclosed herein is a dosage form, comprising
(a) a recombinant protein comprising
   (i) interleukin-12 (IL-12) and
   (ii) a targeting entity, and
(b) a kinase inhibitor
in a pharmaceutically acceptable carrier.

Further disclosed herein is a kit of dosage forms, comprising at least
(a) a first dosage form comprises a recombinant protein comprising
   (i) interleukin-12 (IL-12) and
   (ii) a targeting entity, and
   in a pharmaceutically acceptable carrier and
(b) a second dosage form comprises a kinase inhibitor in a pharmaceutically acceptable carrier.

For example, the pharmaceutical composition, dosage form, combination, or kit according to the above description,
(a) the recombinant protein comprising
   (i) interleukin-12 (IL-12) and
   (ii) a targeting entity, and
(b) the kinase inhibitor
are administered or taken simultaneously.

For example, the pharmaceutical composition, dosage form, combination, or kit according to the above description,
(a) the recombinant protein comprising
   (i) interleukin-12 (IL-12) and
   (ii) a targeting entity, and
(b) the kinase inhibitor
are administered or taken sequentially.

According to one example the kinase inhibitor, like e.g. a JAK kinase inhibitor, preferably Ruxolitinib, is administered or taken before the recombinant protein comprising interleukin-12 (IL-12) and a targeting entity

According to one example of the pharmaceutical composition, dosage form, combination, or kit according to the above description, the targeting entity comprises an anti-fibronectin antibody, or a target binding fragment or derivative thereof.

According to one example of the pharmaceutical composition, dosage form, combination, or kit according to the above description, the targeting entity comprises an antibody binding the extra-domain B (ED-B) of fibronectin, or a target binding fragment or derivative thereof.

Fibronectin is a high-molecular weight (~500-~600 kDa) glycoprotein of the extracellular matrix that binds to membrane-spanning receptor proteins called integrins. Fibronectin also binds to other extracellular matrix proteins such as collagen, fibrin, and heparan sulfate proteoglycans (e.g. syndecans).

Fibronectin exists as a protein dimer, consisting of two nearly identical monomers linked by a pair of disulfide bonds. The fibronectin protein is produced from a single gene, but alternative splicing of its pre-mRNA leads to the creation of several isoforms.

Two types of fibronectin are present in vertebrates:
- soluble plasma fibronectin (formerly called "cold-insoluble globulin", or CIg) is a major protein component of blood plasma (300 µg/ml) and is produced in the liver by hepatocytes.
- insoluble cellular fibronectin is a major component of the extracellular matrix. It is secreted by various cells, primarily fibroblasts, as a soluble protein dimer and is then assembled into an insoluble matrix in a complex cell-mediated process.

Fibronectin plays a major role in cell adhesion, growth, migration, and differentiation, and it is important for processes such as wound healing and embryonic development. Altered fibronectin expression, degradation, and organization has been associated with a number of pathologies, including cancer, arthritis, and fibrosis.

Fibronectin isoform B-FN is one of the best-known markers of angiogenesis (see e.g. WO1997/045544). An extra domain "ED-B" of 91 amino acids is found in the B-FN isoform and is identical in mouse, rat, rabbit, dog and man. B-FN accumulates around neovascular structures in aggressive tumours and other tissues undergoing angiogenesis, such as the endometrium in the proliferative phase and some ocular structures in pathological conditions, but is otherwise undetectable in normal adult tissues.

The extra-domain B (ED-B) of fibronectin is an attractive target for anti-cancer therapy, including the use of immunocytokines as discussed herein, in particular immunocytokines comprising the antibody L19 as discussed herein.

L19 is a human monoclonal scFv specific alternatively spliced ED-B domain of fibronectin and has been previously described (WO1999/058570; WO2003/076469, WO2005/023318).

The term "L 19" as used herein means any antibody that binds to EDB Fibronectin or any portion thereof and comprises an amino acid sequence having at least seventy-five percent (75%) identity to one or more of the following amino acid sequences:

| SEQ ID NO | Qualifier |
|---|---|
| 5 | L19 VL |
| 7 | L19 VH |
| 10 | L19 VHCDR1 |
| 11 | L19 VHCDR2 |
| 12 | L19 VHCDR3 |
| 13 | L19 VLCDR1 |
| 14 | L19 VLCDR2 |
| 15 | L19 VLCDR3 |

The term "VH" as used herein means the heavy chain variable domain of an antibody.

The term "VL" as used herein means the light chain variable domain of an antibody.

The terms "CDR1", "CDR2" and "CDR3" as used herein mean the complementarity determining regions within the light chain or heavy chain variable domain of an antibody.

According to several examples, the pharmaceutical composition, dosage form, combination, or kit according to the above description is provided for (the manufacture of a medicament consisting of at least one dosage form for) use in the treatment of a in a human or mammalian patient
(i) being diagnosed for,
(ii) suffering from or
(iii) being at risk of developing
cancer.

This language is deemed to encompass both the swiss type claim language accepted in some countries (in this case, brackets are deemed absent) and EPC2000 language (in this case, brackets and content within the brackets is deemed absent).

According to several examples, the cancer is at least one selected from the group consisting of solid or non-solid cancer, malignant lymphoma, liver cancer, lymphoma, leukaemia (e.g. acute myeloid leukaemia), sarcomas, skin cancer, bladder cancer, breast cancer, uterine cancer, ovarian cancer, prostate cancer, lung cancer, colorectal cancer, cervical cancer, head and neck cancer, oesophageal cancer, pancreatic cancer, renal cancer, stomach cancer and/or cerebral cancer.

According to several examples of the pharmaceutical composition, dosage form, combination, or kit according to the above description, the antibody binding the extra-domain B (ED-B) of fibronectin comprises the complementarity determining regions (CDRs) of the L19 antibody as shown in SEQ ID Nos: 10-15.

According to several examples of the pharmaceutical composition, dosage form, combination, or kit according to the above description, the antibody binding the extra-domain B (ED-B) of fibronectin comprises the L19 VH as shown in SEQ ID NO: 7 and/or the L19 VL as shown in SEQ ID NO: 5.

According to several examples of the pharmaceutical composition, dosage form, combination, or kit according to the above description, the antibody binding the extra-domain B (ED-B) of fibronectin comprises the amino acid sequence of L19 diabody as shown in SEQ ID NO: 20 According to one example of the pharmaceutical composition, dosage form, combination, or kit according to the above description, the kinase inhibitor is a JAK inhibitor.

Janus kinases (JAKs) are multidomain non-receptor tyrosine kinases that have pivotal roles in intracellular signal transduction. The targeting of JAK-associated pathways through the use of JAK inhibitors has rapidly entered the clinical arena for a wide array of disease states, including myeloproliferative neoplasms, rheumatoid arthritis and other immune-mediated arthropathies, numerous inflammatory dermatological disorders, and inflammatory bowel disease.

In humans, the JAK family comprises JAK1 (also known as Janus kinase-1), JAK2 (also known as Janus kinase-2), JAK3 (also known as Janus kinase, leukocyte; JAKL; L-JAK and Janus kinase-3) and TYK2 (also known as protein-tyrosine kinase 2). Irrespective of type, all cytokine receptors are associated with one or more of the JAKs to facilitate signal transduction

The JAK proteins range in size from 120 to 140 kDa and comprise seven conserved JAK homology (JH) domains; one of these is a functional catalytic kinase domain, and another is a pseudo kinase domain potentially serving a regulatory function and/or serving as a docking site for signal transducer and activator of transcription (STAT).

Recruitment, dimerization, nuclear translocation of STAT results in transcriptional responses. JAK-STAT signaling has a pivotal role in a pleotropic range of systems, including the orchestration and functional capability of immune responses, in particular T-cell polarization, control of hematopoiesis and inflammation, adipogenesis, and growth.

The essential role of JAKs in cytokine signaling and, therefore in inflammatory disorders, such as autoimmune disease and neoplasms, has led to the discovery of therapeutics drugs inhibiting JAK signaling pathway.

Within hematology, the largest area of clinical expansion for JAK inhibitors has been in the myeloproliferative neoplasm field.

The JAK2 Val617Phe mutation, leading to constitutive activation, is located in the JH2 domain and is found in approximately 60% of individuals with myelofibrosis, 50-60% with essential thrombocythemia, and 97-98% with polycythemia vera. Furthermore, irrespective of JAK2 mutational status, many myeloproliferative neoplasm disorders are characterized by upregulated JAK-STAT signaling via mutations in additional canonical genes, such as CALR. In the oncology field, many solid tumours have aberrant JAK signaling as part of a pro-survival phenotype and to facilitate tumour migration.

There has been rapid clinical development of JAK inhibitors and, in 2011, ruxolitinib (a JAK1 and JAK2 inhibitor; Novartis) was the first JAK inhibitor to be approved for use in myelofibrosis by the US Food and Drug Administration (FDA) and European Medicines Agency (EMA). Currently licensed JAK inhibitors also include tofacitinib (Pfizer), fedratinib (Celgene), upadacitinib (AbbVie), peficitinib (Astellas), and baricitinib (Eli Lilly). More novel JAK inhibitor compounds are in advanced development, such as ritlecitinib (previously known as PF-06651600; Pfizer), which acts as a potent inhibitor of JAK3 and tyrosine-protein kinase Tec (TEC) family kinase members (i.e., BTK, BMX, ITK, RLK, and TEC). Clinically relevant selected agents are shown in Table 1.

**Table 1. Drug inhibitors of JAK family members**

| | |
|---|---|
| • | Ruxolitinib (JAK1 and JAK2 >JAK3 >TYK2) |
| • | Baricitinib (JAK1 and JAK2, and moderate activity against TYK2) |
| • | Tofacitinib (JAK3 >JAK2 >JAK1) |
| • | Fedratinib (JAK2) |
| • | Momelotinib (JAK1 and JAK2) |
| • | Pacritinib (JAK2 >JAK1 and JAK3) |
| • | Fligotinib (JAK1 >JAK2 >JAK3 and TYK2) |
| • | Upadacitinib (JAK1 >JAK2 and JAK3) |
| • | Itacitinib (JAK1) |
| • | Decernotinib (JAK3) |
| • | Peficitinib (all JAK proteins) |
| • | Deucravacitinib (BMS-986165; TYK2) |
| • | Abrocitinib (JAK1) |
| • | NDI-031301 (TYK2) |
| • | Ritlecitinib (PF-06651600; JAK3) |

According to one example of the pharmaceutical composition, dosage form, combination, or kit according to the above description, the JAK inhibitor may be Ruxolitinib.

Ruxolitinib, in particular, is a small molecule JAK inhibitor that is used in the treatment of intermediate or high-risk myelofibrosis and resistant forms of polycythemia vera and graft-vs-host disease. Ruxolitinib is associated with transient and usually mild elevations in serum aminotransferase during therapy and to rare instances of self-limited, clinically apparent idiosyncratic acute liver injury as well as to cases of reactivation of hepatitis B in susceptible individuals.

Ruxolitinib is an orally bioavailable JAK inhibitor with potential antineoplastic and immunomodulating activities. Ruxolitinib specifically binds to and inhibits protein tyrosine kinases JAK 1 and 2, which may lead to a reduction in inflammation and an inhibition of cellular proliferation.

Ruxolitinib is a pyrazole substituted at position 1 by a 2-cyano-1-cyclopentylethyl group and at position 3 by a pyrrolo[2,3-d]pyrimidin-4-yl group. It is used as the phosphate salt for the treatment of patients with intermediate or high-risk myelofibrosis, including primary myelofibrosis, post-polycythemia vera myelofibrosis and post-essential thrombocythemia myelofibrosis.

### EXAMPLES

### Example 1: Preparation of the combination partners

### 1.1. Ruxolitinib preparation

Lyophilized Ruxolitinib was initially dissolved in 100% DMSO. For in vivo administration, Ruxolitinib was prepared at a dose of 75 mg/kg in 5% DMSO + 5% Tween-20 in deionized water.

### 1.2. Protein expression and characterization

The L19-IL12 cytokine used in the present experiments, is a fusion protein consisting of the anti-EDB L19 antibody in single chain diabody format fused to a single-chain of murine IL-12 in which the beta p40 and alpha p35 subunits of IL-12 are linked by the 15-amino-acid linker (GGGGS)₃. The cloning of this protein has been described by Puca et al. Int J Cancer (2020), 146, 2518-2530.

The product was purified from the cell culture medium by affinity chromatography using a Protein A affinity column. After dialyses into PBS pH 7.4, the quality of the protein was assessed by SDS-PAGE and by Size-exclusion chromatography on a Superdex 200 Increase 10/300 GL column mounted on an ÄKTA FPLC.

### 1.3. RESULTS

The protein was pure and ran at the correct molecular weight.

### Example 2: Therapy experiments

### 2.1. Preparation of the tumor cells

MC-38 cells, derived from C57BL/6 murine colon adenocarcinoma cells were cultured in DMEM medium supplemented with Fetal Bovine Serum (10%) and antibiotic-antimycotic (1%) following the supplier's protocol and kept in culture for no longer than 10 passages with a confluence lower than 90%. Eight-week-old female C57BL/6 mice were used for the experiment.

### 2.2. Tumor implantation

MC-38 cells were grown to 80% confluence and detached with Trypsin-EDTA 0.05%. Cells were washed, counted and re-suspended in HBSS to a final concentration of 1 × 10⁷ cells ml⁻¹. Aliquots of 1 × 10⁶ cells (100 µl of the suspension) were injected subcutaneously in the right flank of each animal. Tumor volume was determined with the following formula: (length × width² × 0.5) measured with a caliper.

### 2.3. Therapy experiments

When tumors reached an average of about 50-100 mm³, MC-38 tumor-bearing mice were randomized, and different treatments were started. Eight different groups (4/5 mice per group) received the following treatments:
(i) i.v. vehicle on day 8, 11 and 14 after tumor implantation
(ii) s.c. Ruxolitinib (75 mg/kg) on day 8, 11 and 14 after tumor implantation
(iii) i.v. L19-IL12 (0.6 mg/kg) on day 8, 11 and 14 after tumor implantation
(iv) i.v. L19-IL12 (0.9 mg/kg) on day 8, 11 and 14 after tumor implantation
(v) i.v. L19-IL12 (1.2 mg/kg) on day 8, 11 and 14 after tumor implantation
(vi) s.c Ruxolitinib (75 mg/kg) and i.v. L19-IL12 (0.6 mg/kg) on day 8, 11 and 14 after tumor implantation
(vii) s.c. Ruxolitinib (75 mg/kg) and i.v. L19-IL12 (0.9 mg/kg) on day 8, 11 and 14 after tumor implantation
(viii) s.c. Ruxolitinib (75 mg/kg) and i.v. L19-IL12 (1.2 mg/kg) on day 8, 11 and 14 after tumor implantation

Ruxolitinib was always administered 10 minutes prior the subsequent administration of L19-IL12 at the three different dose levels. Each group was injected into the lateral tail vein every 72 hours for three times.

Efficacy of the different treatments was monitored by daily tumour volume measurement using the formula: tumor size = (length (mm) x width² (mm))/2.

Toxicity of the different treatments was monitored by daily body weighing and general appearance of the animals.

### 2.4. RESULTS

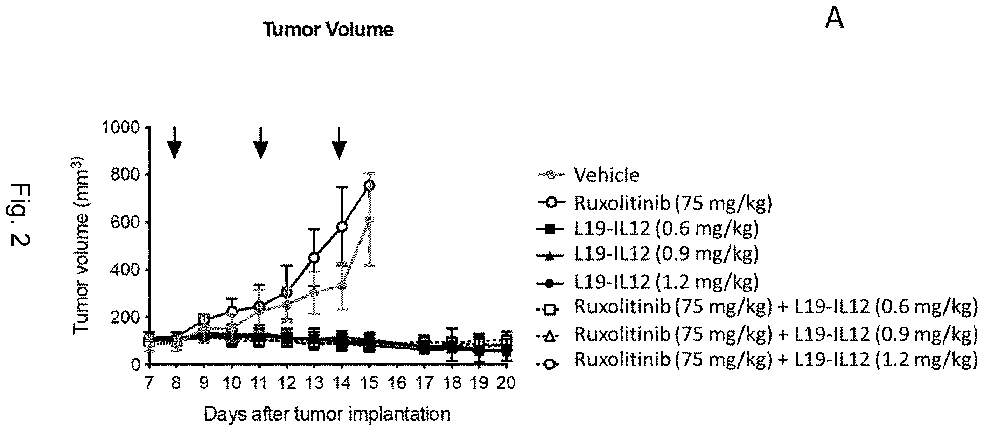

### 2.4.1. Therapy (Fig. 2a)

L19-IL12 has effectively prevented tumor growth both in monotherapy or in combination with Ruxolitinib at each of the three dose levels tested in the experiment.

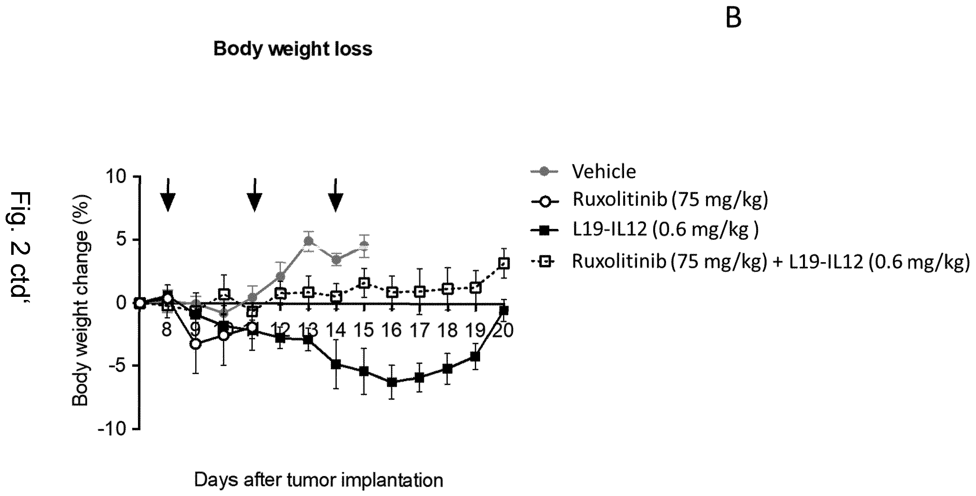

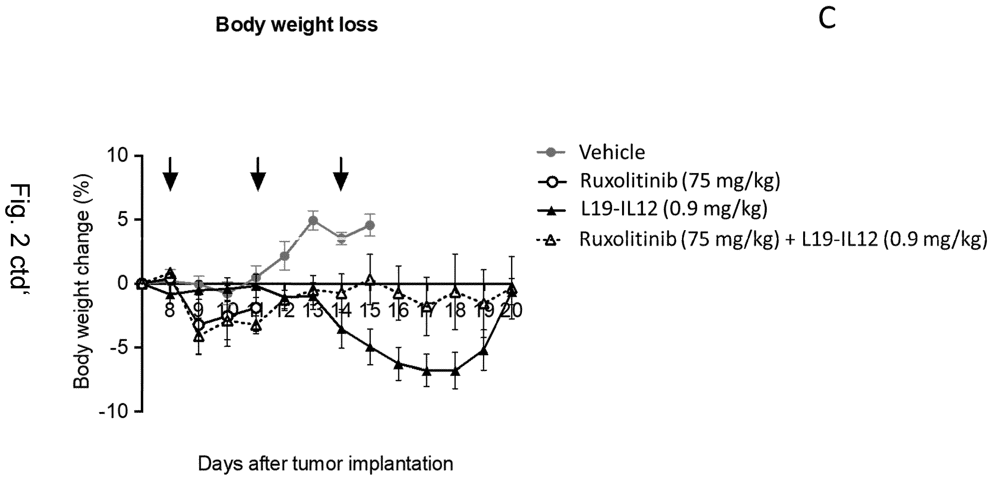

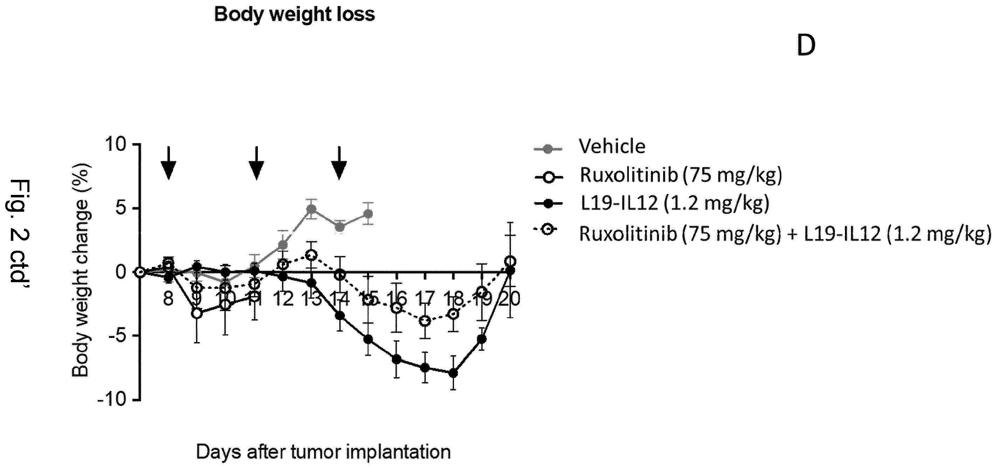

### 2.4.2. Toxicity (Fig. 2b, 2c, 2d)

As expected, the administration of L19-IL12 monotherapy caused a weight loss at each of the three doses tested in the experiments, with higher doses (1.2 and 0.9 mg/kg) causing a more severe toxicity as compared to the lowest dose (0.6 mg/kg). The toxicity effect was more evident between days 14-18.

However, when L19-IL12 was combined with Ruxolitinib the weight loss was negligible at the lower doses (0.6 mg/kg and 0.9 mg/kg) and did not exceed 5% of weight loss at the highest dose (1.2 mg/kg).

Therefore, the combination of L19-IL12 with Ruxolitinib has significantly reduced the toxicity of IL-12.

### Example 3: In vitro bioactivity assay.

### 3.1. In vitro IFN-γ release

Human NK-92 cells were subjected to an IFN-γ release assay upon incubation with different inhibitors. Baricitinib (HY-15315), tofacitinib (HY-40354), upadacitinib (HY-19569) and fedratinib (HY-10409) were purchased from MedChemExpress LLC. Ruxolitinib (S1378) and dexamethasone (S1322) were purchased from Selleckchem. Prior to the assay, NK-92 cells were starved for 4h in plain RPMI medium. Cells were then resuspended at a density of 1 x 10⁶ cells/mL in complete RPMI and 100µl of the cell suspension was incubated with increasing concentrations of inhibitors in the presence of 10ng/mL L19-hIL12. After 24 hours, the levels of IFN-γ in the culture supernatants were quantified by a sandwich enzyme-linked immunosorbent assay (ELISA) using a commercial kit (Biolegend).

### 3.2 RESULTS

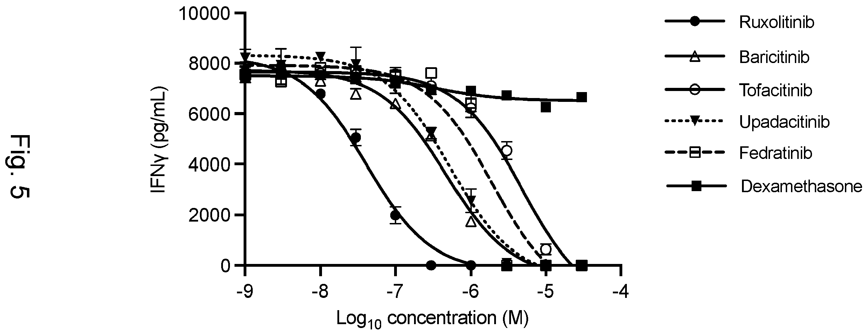

The five JAK inhibitors were benchmarked against Dexamethasone a general antiinflammatory molecule which is commonly used as pre-medication for cytokine release syndrome (CRS) in the clinic. Surprisingly all JAK inhibitors and in particular Ruxolitinib, performed better than Dexamethasone in reducing the production and release of IFN-γ (Figure 5).

### Example 4: Therapy experiments with high dose L19-IL2

Preparation of the tumor cells and tumor implantation have been done as described above in chapters 2.1 and 2.2.

### 4.1. Therapy experiments

When tumors reached an average of about 50-100 mm³, MC-38 tumor-bearing mice were randomized, and different treatments were started. Eight different groups (4/5 mice per group) received the following treatments:
(i) i.v. vehicle on day 8, 11 and 14 after tumor implantation
(ii) s.c. Ruxolitinib (75 mg/kg) on day 8, 11 and 14 after tumor implantation
(iii) i.v. L19-IL12 (2.4mg/kg) on day 8, 11 and 14 after tumor implantation
(iv) i.v. L19-IL12 (3mg/kg) on day 8, 11 and 14 after tumor implantation
(v) s.c Ruxolitinib (75 mg/kg) and i.v. L19-IL12 (2.4mg/kg) on day 8, 11 and 14 after tumor implantation
(vi) s.c. Ruxolitinib (75 mg/kg) and i.v. L19-IL12 (3mg/kg) on day 8, 11 and 14 after tumor implantation

Ruxolitinib was always administered 10 minutes prior the subsequent administration of L19-IL12 at the two different dose levels. Each group was injected into the lateral tail vein every 72 hours for three times. Efficacy of the different treatments was monitored by daily tumour volume measurement using the formula: tumor size = (length (mm) x width² (mm))/2.

Toxicity of the different treatments was monitored by daily body weighing and general appearance of the animals.

### 4.2. RESULTS

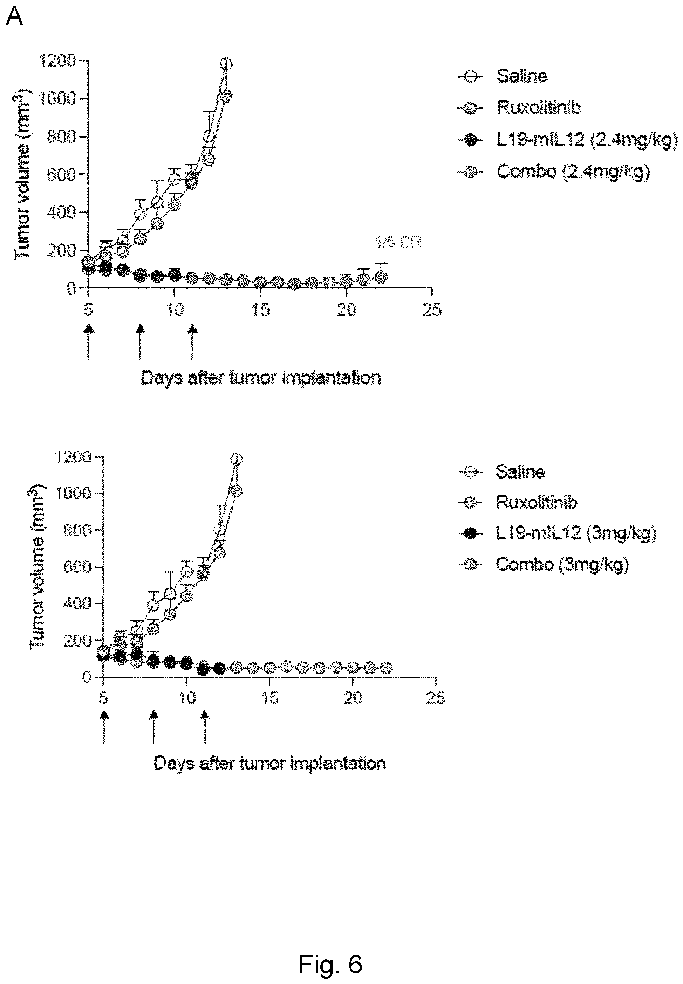

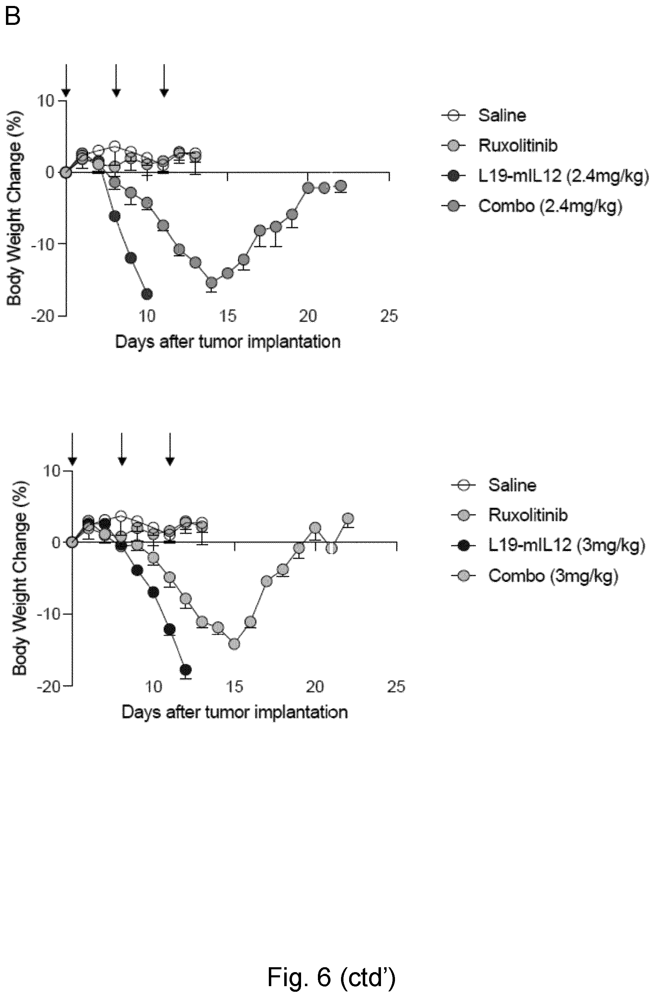

### 4.2.1. Therapy (Fig. 6)

L19-IL12 has effectively prevented tumor growth both in monotherapy or in combination with Ruxolitinib at the two high dose levels tested in the experiment.

### 2.4.2. Toxicity (Fig. 6 continued)

The administration of L19-IL12 monotherapy caused a severe (>15%) weight loss of at the two high dose levels tested in the experiments, which resulted in the sacrifice of the mice for ethical reasons.

However, when L19-IL12 was combined with Ruxolitinib the weight loss never exceeded 15% and returned to normality on around day 20 since start of treatment.

Therefore, even at high doses of L19-IL12 the combination with Ruxolitinib has significantly reduced the toxicity of IL-12.

### Summary of results

(i) Toxicity of an immunocytokine comprising IL12 in rodents starts 48/72 hours after the second injection (Puca et al. Int J Cancer (2020), 146, 2518-2530).
(ii) The blood half-life of Ruxolitinib in rodents is known to be less than 1 hour after injection and - in any case - it is completely washed out within 24 hours (EMA/465846/2012).
(iii) Still, the combination of L19-IL12 with a kinase inhibitor like Ruxolitinib has significantly reduced the toxicity of IL-12.
(iv) The fact that by the time the toxicity of IL-12 starts the kinase inhibitor is no longer present in blood is surprising.

### References

Puca et al. Int J Cancer (2020), 146, 2518-2530
Neri D, Bicknell R. Tumour vascular targeting. Nat Rev Cancer. 2005 Jun;5(6):436-46
Pasche et al. (2011) J Biotechnology, 154, 84-92)
Kaspar et al. (2007) Cancer Res, 67, 4940-4948

### Sequences

The following sequences form part of the disclosure of the present application. A WIPO ST 25 compatible electronic sequence listing will be provided with the nonprovisional application, too. For the avoidance of doubt, if discrepancies exist between the sequences in the following table and the electronic sequence listing, the sequences in this table shall be deemed to be the correct ones.

| SEQ ID NO | Qualifier | Sequence |
|---|---|---|
| 1 | p40 subunit of human IL-12 | IWELKKDVYVVELDWYPDAPGEMVVLTCDTPEEDGITWTLDQSSEV LGSGKTLTIQVKEFGDAGQYTCHKGGEVLSHSLLLLHKKEDGIWST DILKDQKEPKNKTFLRCEAKNYSGRFTCWWLTTISTDLTFSVKSSR GSSDPQGVTCGAATLSAERVRGDNKEYEYSVECQEDSACPAAEESL PIEVMVDAVHKLKYENYTSSFFIRDIIKPDPPKNLQLKPLKNSRQV EVSWEYPDTWSTPHSYFSLTFCVQVQGKSKREKKDRVFTDKTSATV ICRKNASISVRAQDRYYSSSWSEWASVPCS |
| 2 | linker between p40 subunit and p35 subunit both human and murine | GGGGSGGGGSGGGGS |
| 3 | p35 subunit of human IL-12 | RNLPVATPDPGMFPCLHHSQNLLRAVSNMLQKARQTLEFYPCTSEE IDHEDITKDKTSTVEACLPLELTKNESCLNSRETSFITNGSCLASR KTSFMMALCLSSIYEDLKMYQVEFKTMNAKLLMDPKRQIFLDQNML AVIDELMQALNFNSETVPQKSSLEEPDFYKTKIKLCILLHAFRIRA VTIDRVMSYLNAS |
| 4 | linker between human IL-12 and VH chain of antibody or antibody fragment | GSADGGSSAGGSDAG |
| 5 | L19 VL | |
| 6 | linker between VH and VL | GSSGG |
| 7 | L19 VH | |
| 8 | linker between the two single-chain Fv's forming the single chain diabody | SSSSGSSSSGSSSSG |
| 9 | Full length L19-human IL12 | IWELKKDVYVVELDWYPDAPGEMVVLTCDTPEEDGITWTLDQSSEV LGSGKTLTIQVKEFGDAGQYTCHKGGEVLSHSLLLLHKKEDGIWST DILKDQKEPKNKTFLRCEAKNYSGRFTCWWLTTISTDLTFSVKSSR GSSDPQGVTCGAATLSAERVRGDNKEYEYSVECQEDSACPAAEESL PIEVMVDAVHKLKYENYTSSFFIRDIIKPDPPKNLQLKPLKNSRQV EVSWEYPDTWSTPHSYFSLTFCVQVQGKSKREKKDRVFTDKTSATV ICRKNASISVRAQDRYYSSSWSEWASVPCSGGGGSGGGGSGGGGSR NLPVATPDPGMFPCLHHSQNLLRAVSNMLQKARQTLEFYPCTSEEI DHEDITKDKTSTVEACLPLELTKNESCLNSRETSFITNGSCLASRK TSFMMALCLSSIYEDLKMYQVEFKTMNAKLLMDPKRQIFLDQNMLA VIDELMQALNFNSETVPQKSSLEEPDFYKTKIKLCILLHAFRIRAV TIDRVMSYLNASGSADGGSSAGGSDAGEVQLLESGGGLVQPGGSLR LSCAASGFTFSSFSMSWVRQAPGKGLEWVSSISGSSGTTYYADSVK GRFTISRDNSKNTLYLQMNSLRAEDTAVYYCAKPFPYFDYWGQGTL VTVSSGSSGGEIVLTQSPGTLSLSPGERATLSCRASQSVSSSFLAW YQQKPGQAPRLLIYYASSRATGIPDRFSGSGSGTDFTLTISRLEPE DFAVYYCQQTGRIPPTFGQGTKVEIKSSSSGSSSSGSSSSGEVQLL ESGGGLVQPGGSLRLSCAASGFTFSSFSMSWVRQAPGKGLEWVSSI SGSSGTTYYADSVKGRFTISRDNSKNTLYLQMNSLRAEDTAVYYCA KPFPYFDYWGQGTLVTVSSGSSGGEIVLTQSPGTLSLSPGERATLS CRASQSVSSSFLAWYQQKPGQAPRLLIYYASSRATGIPDRFSGSGS GTDFTLTISRLEPEDFAVYYCQQTGRIPPTFGQGTKVEIK |
| 10 | L19 VHCDR1 | SFSMS |
| 11 | L19 VHCDR2 | SISGSSGTTYYADSVKG |
| 12 | L19 VHCDR3 | PFPYFDY |
| 13 | L19 VLCDR1 | RASQSVSSSFLA |
| 14 | L19 VLCDR2 | YASSRAT |
| 15 | L19 VLCDR3 | QQTGRIPPT |
| 16 | p40 subunit of murine IL-12 | MWELEKDVYVVEVDWTPDAPGETVNLTCDTPEEDDITWTSDQRHGV IGSGKTLTITVKEFLDAGQYTCHKGGETLSHSHLLLHKKENGIWST EILKNFKNKTFLKCEAPNYSGRFTCSWLVQRNMDLKFNIKSSSSSP DSRAVTCGMASLSAEKVTLDQRDYEKYSVSCQEDVTCPTAEETLPI ELALEARQQNKYENYSTSFFIRDIIKPDPPKNLQMRPLKNSQVEVS WEYPDSWSTPHSYFSLKFFVRIQRKKEKMKETEEGCNQKGAFLVER TSTEVQCKGGNVCVQAQDRYYNSSCSKWACVPCRVRS |
| 17 | p35 subunit of murine IL-12 | RVIPVSGPARCLSQSRNLLKTTDDMVKTAREKLKHYSCTAEDIDHE DITRDQTSTLKTCLPLELHKNESCLATRETSSTTRGSCLPPQKTSL MMTLCLGSIYEDLKMYQTEFQAINAALQNHNHQQIILDKGMLVAID ELMQSLNHNGETLRQKPPVGEADPYRVKMKLCILLHAFSTRVVTIN RVMGYLSSA |
| 18 | linker between murine IL-12 and VH chain of antibody or antibody fragment | GSADG |
| 19 | Full length L19-murine IL12 | MWELEKDVYVVEVDWTPDAPGETVNLTCDTPEEDDITWTSDQRHGV IGSGKTLTITVKEFLDAGQYTCHKGGETLSHSHLLLHKKENGIWST EILKNFKNKTFLKCEAPNYSGRFTCSWLVQRNMDLKFNIKSSSSSP DSRAVTCGMASLSAEKVTLDQRDYEKYSVSCQEDVTCPTAEETLPI |
| | | ELALEARQQNKYENYSTSFFIRDIIKPDPPKNLQMRPLKNSQVEVS WEYPDSWSTPHSYFSLKFFVRIQRKKEKMKETEEGCNQKGAFLVER TSTEVQCKGGNVCVQAQDRYYNSSCSKWACVPCRVRSGGGGSGGGG SGGGGSRVIPVSGPARCLSQSRNLLKTTDDMVKTAREKLKHYSCTA EDIDHEDITRDQTSTLKTCLPLELHKNESCLATRETSSTTRGSCLP PQKTSLMMTLCLGSIYEDLKMYQTEFQAINAALQNHNHQQIILDKG MLVAIDELMQSLNHNGETLRQKPPVGEADPYRVKMKLCILLHAFST RVVTINRVMGYLSSAGSADGEVQLLESGGGLVQPGGSLRLSCAASG FTFSSFSMSWVRQAPGKGLEWVSSISGSSGTTYYADSVKGRFTISR DNSKNTLYLQMNSLRAEDTAVYYCAKPFPYFDYWGQGTLVTVSSGS SGGEIVLTQSPGTLSLSPGERATLSCRASQSVSSSFLAWYQQKPGQ APRLLIYYASSRATGIPDRFSGSGSGTDFTLTISRLEPEDFAVYYC QQTGRIPPTFGQGTKVEIKSSSSGSSSSGSSSSGEVQLLESGGGLV QPGGSLRLSCAASGFTFSSFSMSWVRQAPGKGLEWVSSISGSSGTT YYADSVKGRFTISRDNSKNTLYLQMNSLRAEDTAVYYCAKPFPYFD YWGQGTLVTVSSGSSGGEIVLTQSPGTLSLSPGERATLSCRASQSV SSSFLAWYQQKPGQAPRLLIYYASSRATGIPDRFSGSGSGTDFTLT ISRLEPEDFAVYYCQQTGRIPPTFGQGTKVEIK |
| 20 | L19 diabody | EVQLLESGGGLVQPGGSLRLSCAASGFTFSSFSMSWVRQAPGKGLE WVSSISGSSGTTYYADSVKGRFTISRDNSKNTLYLQMNSLRAEDTA VYYCAKPFPYFDYWGQGTLVTVSSGSSGGEIVLTQSPGTLSLSPGE RATLSCRASQSVSSSFLAWYQQKPGQAPRLLIYYASSRATGIPDRF SGSGSGTDFTLTISRLEPEDFAVYYCQQTGRIPPTFGQGTKVEIK |
| 21 | alternative linker between human IL-12 and VH chain of antibody or antibody fragment | GGGSGGGGSSGGGGS |
| 22 | | GGGAKGGGGKAGGGS |
| 23 | | GGGGSGGGGEGGGGS |
| 24 | | GGGGDGGGGDGGGGS |
| 25 | | APAPAPAPAPAPAP |
| 26 | | APAPAPAPAPAP |
| 27 | | AEAAAKEAAAKEAAAKA |

## Claims

1. A combination comprising at least
(a) a recombinant protein comprising
(i) interleukin-12 (IL-12) and
(ii) a targeting entity which comprises an antibody binding the extra-domain B (ED-B) of fibronectin, or a target binding fragment, and
(b) a kinase inhibitor,
wherein the kinase inhibitor is the JAK inhibitor Ruxolitinib, and
wherein the targeting entity is an antibody, or a fragment thereof able to bind to a given target with high specificity and affinity.

2. The combination according to claim 1, wherein
(a) the recombinant protein comprising
(i) interleukin-12 (IL-12) and
(ii) a targeting entity which comprises an antibody binding the extra-domain B (ED-B) of fibronectin, or a target binding fragment, and
(b) the kinase inhibitor
are administered or taken simultaneously.

3. The combination according to claim 1, wherein
(a) the recombinant protein comprising
(i) interleukin-12 (IL-12) and
(ii) a targeting entity which comprises an antibody binding the extra-domain B (ED-B) of fibronectin, or a target binding fragment, and
(b) the kinase inhibitor
are administered or taken sequentially.

4. The combination according to any of claims 1 - 3, wherein the antibody binding the extra-domain B (ED-B) of fibronectin comprises
a) the complementarity determining regions (CDRs) of the L19 antibody as shown in SEQ ID Nos: 10-15, and/or
b) the L19 VH as shown in SEQ ID NO: 7 and/or the L19 VL as shown in SEQ ID NO: 5.

5. The combination according to any of claims 1-4, wherein the antibody binding the extra-domain B (ED-B) of fibronectin comprises the amino acid sequence of L19 whose CDRs are shown in SEQ ID NO: 10-15.

6. The combination according to any of claims 1 - 5 for use in the treatment of a in a human or mammalian patient
(i) being diagnosed for,
(ii) suffering from or
(iii) being at risk of developing
cancer.

7. The combination for use according to claim 6, wherein the cancer is at least one selected from the group consisting of solid or non-solid cancer, malignant lymphoma, liver cancer, lymphoma, leukaemia (e.g. acute myeloid leukaemia), sarcomas, skin cancer, bladder cancer, breast cancer, uterine cancer, ovarian cancer, prostate cancer, lung cancer, colorectal cancer, cervical cancer, head and neck cancer, oesophageal cancer, pancreatic cancer, renal cancer, stomach cancer and/or cerebral cancer.

## Patentansprüche

1. Kombination, aufweisend mindestens
(a) ein rekombinantes Protein, aufweisend
(i) Interleukin-12 (IL-12) und
(ii) eine Zielentität, die einen Antikörper, der die Extra-Domäne B (ED-B) von Fibronektin bindet, oder ein zielbindendes Fragment, aufweist, und
(b) einen Kinase-Inhibitor,
wobei der Kinase-Inhibitor der JAK-Inhibitor Ruxolitinib ist, und
wobei die Zielentität ein Antikörper oder ein Fragment davon ist, das in der Lage ist, an ein bestimmtes Ziel mit hoher Spezifität und Affinität zu binden.

2. Kombination nach Anspruch 1, wobei
(a) das rekombinante Protein aufweisend
(i) Interleukin-12 (IL-12) und
(ii) eine Zielentität, die einen Antikörper, der die Extra-Domäne B (ED-B) von Fibronektin bindet, oder ein zielbindendes Fragment, aufweist, und
(b) der Kinase-Inhibitor
gleichzeitig verabreicht oder eingenommen werden.

3. Kombination nach Anspruch 1, wobei
(a) das rekombinante Protein aufweisend
(i) Interleukin-12 (IL-12) und
(ii) eine Zielentität, die einen Antikörper, der die Extra-Domäne B (ED-B) von Fibronektin bindet, oder ein zielbindendes Fragment, aufweist, und
(b) der Kinase-Inhibitor
nacheinander verabreicht oder eingenommen werden.

4. Kombination nach einem der Ansprüche 1 bis 3, wobei der Antikörper, der die Extra-Domäne B (ED-B) von Fibronektin bindet, aufweist
a) die komplementaritätsbestimmenden Regionen (CDRs) des L19-Antikörpers, wie in SEQ ID Nrn: 10-15 gezeigt, und/oder
b) die L19 VH, wie in SEQ ID Nr: 7 gezeigt, und/oder die L19 VL, wie in SEQ ID Nr: 5 gezeigt.

5. Kombination nach einem der Ansprüche 1-4, wobei der Antikörper, der die Extra-Domäne B (ED-B) von Fibronektin bindet, die Aminosäuresequenz von L19 aufweist, deren CDRs in SEQ ID Nr: 10-15 gezeigt sind.

6. Kombination nach einem der Ansprüche 1 bis 5 zur Verwendung bei der Behandlung eines menschlichen oder Säugetier-Patienten
(i) diagnostiziert für
(ii) leidend unter, oder
(iii) mit dem Risiko der Entwicklung von
Krebs.

7. Kombination zur Verwendung nach Anspruch 6, wobei der Krebs mindestens einer ist, ausgewählt aus der Gruppe bestehend aus solidem oder nicht solidem Krebs, malignem Lymphom, Leberkrebs, Lymphom, Leukämie (z.B. akuter myeloischer Leukämie), Sarkomen, Hautkrebs, Blasenkrebs, Brustkrebs, Gebärmutterkrebs, Eierstockkrebs, Prostatakrebs, Lungenkrebs, kolorektalem Krebs, Gebärmutterhalskrebs, Kopf- und Halskrebs, Speiseröhrenkrebs, Bauchspeicheldrüsenkrebs, Nierenkrebs, Magenkrebs und/oder Hirnkrebs.

## Revendications

1. Combinaison comprenant au moins
(a) une protéine recombinante comprenant
(i) de l'interleukine-12 (IL-12) et
(ii) une entité de ciblage qui comprend un anticorps se liant au domaine extra B (ED-B) de la fibronectine, ou un fragment de liaison à la cible, et
(b) un inhibiteur de kinase,
dans laquelle l'inhibiteur de kinase est l'inhibiteur de JAK Ruxolitinib, et
dans laquelle l'entité de ciblage est un anticorps, ou un fragment de celui-ci capable de se lier à une cible donnée avec une spécificité et une affinité élevées.

2. Combinaison selon la revendication 1, dans laquelle
(a) la protéine recombinante comprenant
(i) l'interleukine-12 (IL-12) et
(ii) une entité de ciblage qui comprend un anticorps se liant au domaine extra B (ED-B) de la fibronectine, ou un fragment de liaison à la cible, et
(b) l'inhibiteur de kinase
sont administrés ou pris de façon simultanée.

3. Combinaison selon la revendication 1, dans laquelle
(a) la protéine recombinante comprenant
(i) l'interleukine-12 (IL-12) et
(ii) une entité de ciblage qui comprend un anticorps se liant au domaine extra B (ED-B) de la fibronectine, ou un fragment de liaison à la cible, et
(b) l'inhibiteur de kinase
sont administrés ou pris de façon séquentielle.

4. Combinaison selon l'une quelconque des revendications 1 à 3, dans laquelle l'anticorps se liant au domaine extra B (ED-B) de la fibronectine comprend
a) les régions déterminant la complémentarité (CDR) de l'anticorps L19 telles que représentées dans les SEQ ID n° : 10 à 15, et/ou
b) la VH de L19 telle que représentée dans la SEQ ID n° : 7 et/ou la VL de L19 telle que représentée dans la SEQ ID n° : 5.

5. Combinaison selon l'une quelconque des revendications 1 à 4, dans laquelle l'anticorps se liant au domaine extra B (ED-B) de la fibronectine comprend la séquence d'acides aminés de L19 dont les CDR sont représentées dans les SEQ ID n° : 10 à 15.

6. Combinaison selon l'une quelconque des revendications 1 à 5 pour utilisation dans le traitement chez un patient humain ou mammifère
(i) ayant reçu un diagnostic de,
(ii) souffrant de, ou
(iii) présentant un risque de développer un cancer.

7. Combinaison pour utilisation selon la revendication 6, dans laquelle le cancer est au moins un cancer choisi dans le groupe constitué par un cancer solide ou non solide, un lymphome malin, un cancer du foie, un lymphome, une leucémie (par exemple une leucémie myéloïde aiguë), des sarcomes, un cancer de la peau, un cancer de la vessie, un cancer du sein, un cancer de l'utérus, un cancer de l'ovaire, un cancer de la prostate, un cancer du poumon, un cancer colorectal, un cancer du col de l'utérus, un cancer de la tête et du cou, un cancer de l'œsophage, un cancer du pancréas, un cancer du rein, un cancer de l'estomac et/ou un cancer cérébral.
